# EUROPEAN PATENT SPECIFICATION

(11) **EP 2 352 506 B1**
(45) Date of publication and mention of the grant of the patent: **20.07.2016**
(21) Application number: 10810813.5
(22) Date of filing: 07.12.2010
(51) Int. Cl.: A61K 36/60, A61K 36/355, A61P 29/00, A61P 11/06

(54) **ANTI-INFLAMMATORY PHARMACEUTICAL COMPOSITION COMPRISING EXTRACTS FROM BROUSSONETIA PAPYRIFERA AND LONICERA JAPONICA**
ENTZÜNDUNGSHEMMENDE PHARMAZEUTISCHE ZUSAMMENSETZUNG MIT EXTRAKTEN AUS BROUSSONETIA PAPYRIFERA UND LONICERA JAPONICA
COMPOSITION PHARMACEUTIQUE ANTI-INFLAMMATOIRE CONTENANT DES EXTRAITS DE BROUSSONETIA PAPYRIFERA ET DE LONICERA JAPONICA

(30) Priority: 07.12.2009 KR 20090120711
(43) Date of publication of application: 10.08.2011
(73) Proprietor: Pharmaking Co., Ltd., Eumseong-gun, Chungcheongbuk-do 369-852 (KR); KNU-Industry Cooperation Foundation, Gangwon-do 200-701 (KR)
(72) Inventor: KIM, Soon Bae, Seongnam-si, Gyeonggi-do 463-500 (KR); KIM, Gwang Soon, Gyeonggi-do 463-744 (KR); KIM, Wan Bae, Seoul 110-797 (KR); KWAK, Wie Jong, Seoul 137-764 (KR); BANG, Sung Hye, Seoul 120-130 (KR); KIM, Hyun Pyo, Seoul 138-882 (KR); SON, Kun Ho, Gyeongsangbuk-do 760-757 (KR)
(74) Representative: Gulde & Partner
(86) International application number: PCT/KR2010/008710
(87) International publication number: WO 2011/071296

(56) References cited:
- WO-A2-2009/104913
- KR-A- 20020 011 553
- KR-B1- 0 163 813
- KR-B1- 100 672 036
- US-A1- 2007 111 955
- K.H SON ET AL: "Papyriflavonol A, a new prenylated flavonol from Broussonetia papyrifera", FITOTERAPIA, vol. 72, no. 4, 1 May 2001 (2001-05-01), pages 456-458, XP55041034, ISSN: 0367-326X, DOI: 10.1016/S0367-326X(00)00329-4
- KAWAI H ET AL: "IRIDOID GLUCOSIDES FROM LONICERA-JAPONICA THUNB", CHEMICAL & PHARMACEUTICAL BULLETIN, PHARMACEUTICAL SOCIETY OF JAPAN, JP, vol. 36, no. 9, 1 January 1988 (1988-01-01), pages 3664-3666, XP009163735, ISSN: 0009-2363 [retrieved on 1988-09-25]
- YEON SOOK CHI ET AL: "Effects of naturally occurring prenylated flavonoids on enzymes metabolizing arachidonic acid: Cyclooxygenases and lipoxygenases", BIOCHEMICAL PHARMACOLOGY, vol. 62, no. 9, 1 November 2001 (2001-11-01), pages 1185-1191, XP55041154, ISSN: 0006-2952, DOI: 10.1016/S0006-2952(01)00773-0
- JEONG-HO JIN ET AL: "Anti-Inflammatory Activity of the Total Flavonoid Fraction from Broussonetia papyrifera in Combination with Lonicera japonica", BIOMOLECULES AND THERAPEUTICS, vol. 18, no. 2, 30 April 2010 (2010-04-30) , pages 197-204, XP55041204, ISSN: 1976-9148, DOI: 10.4062/biomolther.2010.18.2.197
- J. TAE ET AL.: 'Anti-inflammatory effect of Lonicera japonica in proteinase- activated receptor 2-mediated paw edema' CLINICA CHIMICA ACTA vol. 16, 2003, pages 5 - 171, XP008100484
- S. J. LEE ET AL.: 'Antiinflammatory activity of Lonicera japonica' PHYTOTHERAPY RESEARCH vol. 12, 1998, pages 445 - 447, XP008102966
- L.-W. LIN ET AL.: 'Comparison with various parts of Broussonetia papyrifera as to the antinociceptive and anti-inflammatory activities in rodents' BIOSCIENCE BIOTECHNOLOGY AND BIOCHEMISTRY vol. 72, no. 9, 2008, pages 2377 - 2384, XP008150760
- Z.-J. CHENG ET AL.: 'Broussochalcone A, a potent antioxidant and effective s uppressor of inducible nitric oxide synthase in lipopolysaccharide-activated macrophages' BIOCHEMICAL PHARMACOLOGY vol. 61, 2001, pages 939 - 946, XP001149401

## Description

### Technical Field

The present invention relates to a pharmaceutical composition for preventing, alleviating, or treating inflammations or pains, the pharmaceutical composition including extracts from Broussonetia papyrifera and Lonicera japonica as effective components.

### Background Art

Inflammations are preventative reactions occurring in the body when biological tissues are damaged, and may cause hyperemia, edemas, pyrexia, and pains in a part of the body in response to an external wound, bum, or bacterial infection. An inflammatory signal is generated through a cyclooxygenase (COX) pathway and a lipoxygenase (LOX) pathway, and prostaglandin, leukotriene, thromboxane, etc., are generated through these pathways. Once an inflammatory signal is transferred to a target site in the body, various biological changes occur. For example, a blood vessel near the target site to undergo inflammation expands, thereby allowing more blood to be supplied to the blood vessel so that blood cells needed for an inflammatory reaction, such as neutrophils that consume bacteria, are supplied to the target site. However, if the preventive reaction abnormally and excessively occurs in the body, various inflammatory diseases may develop. Accordingly, drugs that suppress an inflammatory reaction by blocking a pathway for generating an inflammatory signal by hindering generation of an enzyme (for example, COX-1, COX-2, 5-LOX, or 12-LOX) involved in the pathway have been developed.

Meanwhile, in addition to an inflammatory reaction, LOX has been known to be engaged in bronchial asthma, chronic obstructive pulmonary disease (COPD), atopic dermatitis, psoriasis, etc.

Up to now, anti-inflammatory drugs having various mechanisms, for example, NSAID and SAID, have been developed. However, they have side effects and are not effective for fundamentally treating COPD, a chronic inflammation such as an atopic disease, and an allergic disease. Thus, there is a need to develop an effective, stable, and economic pharmaceutical product for suppressing inflammation.

Broussonetia papyrifera (L.) Vent. (Moraceae) grows in China, Japan, and Korea, and a bark of the Broussonetia papyrifera has been used as an anti-inflammatory drug, anti-bronchitis drug, an antitussive agent in Korean traditional medicine. According to one report, a radix of Broussonetia papyrifera has an inhibitory activity on PTP1B and tyrosinase (Chen et al., 2002; Hwang and Lee, 2007), and recently, it was reported that 95% ethanol extract from a radix, a stem, a leaf, and a fruit of Broussonetia papyrifera has antinociceptive and anti-inflammatory activities in the body (Lin et al., 2008). Also, several kinds of prenylated flavonoids including papyriflavonol A and broussochalcone A were successfully isolated from a radix bark of Broussonetia papyrifera (Son et al., 2001).

Lonicera japonica (Thunb.) (Caprifoliaceae) is a shrub that grows winding around other things, is used as an antidote, and is used in treating disorders of the urinary system, high fever, and headaches (Shougakukan, 1985). Lonicera japonica is known as an anti-inflammatory drug and is used in treating upper respiratory tract infections, diabetes, and rheumatic arthritis (Lee et al., 1998). Also, several kinds of components including lonicerosides and iridoids are isolated from the whole plant of Lonicera japonica (Lee et al., 1995; Kwak et al., 2003 and Qian et al., 2007).

### Disclosure of Invention

### Technical Problem

Up to now, anti-inflammatory drugs having various mechanisms have been developed. However, they have side effects, and also, in treating some diseases, they are not effective as a fundamental treatment. Thus, there is a need to develop an effective, stable, and economic pharmaceutical product. Such a pharmaceutical product may be a complex including two or more effective components. However, when materials having anti-inflammatoiy and antinociceptive effects are administered in combination to the body, it is difficult to predict the resultant effect in the body. The purpose of the present invention is to provide a novel anti-inflammatoiy combination composition that has a synergic anti-inflammatory effect when different effective materials are administered in combination.

### Solution to Problem

To achieve the purpose, the inventors of the present invention studied and found that when extracts from Broussonetia papyrifera and Lonicera japonica are used in combination, these extracts have better anti-inflammatory and antinociceptive effects than when used separately, and based on the finding, they developed a composition for preventing, alleviating, or treating inflammations or pains, the composition including extracts from Broussonetia papyrifera and Lonicera japonica as effective components. The extract from Broussonetia papyrifera included in the pharmaceutical composition is a prenylated flavonoid-enriched extract and the extract from Lonicera japonica is an ethanol extract from Lonicera japonica.

### Advantageous Effects of Invention

According to the present invention, a composition including extracts from Broussonetia papyrifera and Lonicera japonica as effective components may be used as an effective anti-inflammatory agent, antiphlogistic agent, or analgesic agent because when the extracts are used in combination, better anti-inflammatory and antinociceptive effects are obtained than they are used separately.

The pharmaceutical composition according to the present invention has a wide therapeutical range and a synergic and strong therapeutic effect due to inclusion of extracts from Broussonetia papyrifera and Lonicera japonica. When the extracts from Broussonetia papyrifera and Lonicera japonica are used in combination, a therapeutic effect is increased. Thus, desired effects may be obtained using a smaller amount than that when the extracts are used separately. Also, use of a smaller amount may lead to fewer side effects or adverse effects.

### Brief Description of Drawings

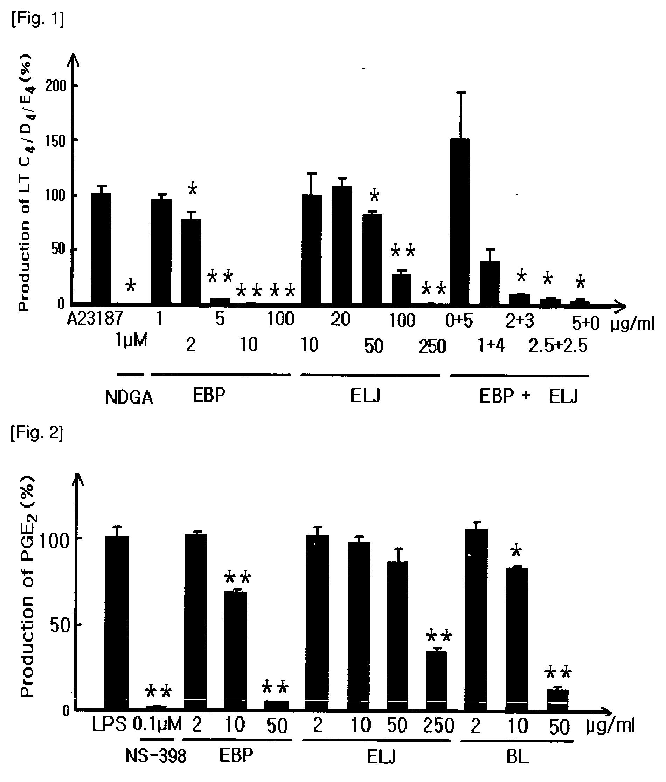

FIG. 1 shows effects of extracts from Broussonetia papyrifera and Lonicera japonica on production of leukotriene from A23187-treated RBL-1 cells mediated by 5-LOX. * and ** respectively indicate a significance of P<0.05 and P<0.01 compared to a control group treated with A23187.
FIG. 2 shows effects of a prenylated flavonoid-enriched extract from Broussonetia papyrifera and/or an ethanol extract from Lonicera japonica on production of PGE2 from LPS-treated RAW 264.7 cells. A LPS-treated group produced 120± 8.0nM PGE2 (basal levels of the respective groups were 1.2± 0.4nM PGE2 and 0.8± 0.3µM NO, respectively). n=3. * and ** respectively indicate a significance of p<0.05 and p<0.01 compared to a LPS-treated control group.

### Mode for the Invention

The present invention relates to a pharmaceutical composition for alleviating or treating inflammations and/or pains, the composition comprising as an effective component an extract from Broussonetia papyrifera or a fraction of the extract and an extract from Lonicera japonica, and a method of treating inflammation and pains by using the pharmaceutical composition.

The present invention is based on a finding that when a prenylated flavonoid-enriched extract from Broussonetia papyrifera and an ethanol extract from Lonicera japonica are administered in combination, excellent synergic anti-inflammatory and analgesic effects occur in in vitro and in vivo tests, compared to when the extracts are administered separately.

The inventors of the present invention prepared a hydrophobic prenylated flavonoid-enriched fraction (EBP) by fractionating an extract from a radix bark of Broussonetia papyrifera by using ethyl acetate and an ethanol extract (ELJ) from a whole plant of Lonicera japonica. EBP strongly inhibits the production of 5-lipoxygenase (5-LOX)mediated leukotriene from A23187-treated rat basophilic leukemia (RBL-1) cells. ELJ has a weak inhibitory activity against the same. However, when EBP and ELJ are used in combination, a strong synergic inhibitory activity is shown (FIG.1).

EBP and ELJ also inhibit production of cyclooxygenase-2-catalyzed PGE₂ (FIG. 2). Regarding arachidonic acid-induced ear edema of a mouse, 5-200 mg/kg of orally administered EBP inhibits the ear edema. A mixture including EBP and ELJ at a weight ratio of 1:1, that is, BL more strongly inhibits the ear edema than EBP or ELJ administered separately(Table 1). Regarding λ-carrageenan-induced paw edema, BL has a strong and synergic anti-inflammatory effect (Table2). In an acetic acid-induced writhing test, BL shows a strong analgesic activity when orally administered in an amount of 50-400 mg/kg (Table 3). Such results show that the tested materials all have an anti-inflammatory activity in vitro and in vivo, and also show that when EBP and ELJ are used in combination, a stronger and synergic anti-inflammatory effect is obtained than when they are used separately.

Based on the experimental results, the present invention provides a pharmaceutical composition for preventing, alleviating, or treating inflammations and/or pains, comprising extracts from Broussonetia papyrifera and Lonicera japonica as effective components.

The present invention also provides a method of preventing, alleviating, or treating inflammations and/or pains, by administering extracts from Broussonetia papyrifera and Lonicera japonica as effective components to the subject.

An extract from Broussonetia papyrifera may be prepared by, for example, extracting using a hydrothermal fluid, an aqueous alcohol solution, or a hydrophobic solvent. In addition, an aqueous alcohol extract may also be extracted using a hydrophobic solvent. However, the extraction method is not limited thereto. An extract from Lonicera japonica may be prepared by, for example, extracting using a hydrothermal fluid, an aqueous alcohol solution, or a hydrophobic solvent. However, the extraction method is not limited thereto. For use in the pharmaceutical composition according to the present invention, a prenylated flavonoid-enriched extract from Broussonetia papyrifera may be used as an extract from a radix bark of Broussonetia papyrifera, and an ethanol extract from Lonicera japonica may be used as an extract from Lonicera japonica. The prenylated flavonoid-enriched extract from Broussonetia papyrifera may be prepared by, for example, extracting Broussonetia papyrifera with ethanol, drying the extract under vacuum condition, and fractionating the extract using ethyl acetate. However, any one of various methods for preparing a prenylated flavonoid-enriched extract may also be used herein. The amount of ethanol used may be in a range of 5 to 100%, and preferably, 50-100%.

A weight ratio of the prenylated flavonoid-enriched extract from Broussonetia papyrifera to the ethanol extract from Lonicera japonica used as an effective component in the pharmaceutical composition according to the present invention may be in a range of 1: 0.1 to 1:10, preferably 1:0.5 to 1:4, more preferably 1:1.

The pharmaceutical composition according to the present invention may be administered either as a formulation that is suitable for oral administration according to a method commonly employed in the pharmaceutical field and prepared in an administration unit or as an injection formulation. Examples of a formulation that is suitable for oral administration are hard and soft capsule, tablet, suspension, syrup, powder, sustained-release formulation, enteric coated formulation, granule, oleosaccharum, fine granule, pill, extract, liquid formulation, aromatic water formulation, emulsion, elixir, fluid extracts, precipitation formulation, tincture, spirits, etc.

Such orally administered formulations may include, in addition to two or more pharmaceutically effective components, one or more pharmaceutically inactive commonly used carrier. Examples of the pharmaceutically inactive carrier are an filler, such as starch, lactose, carboxymethylcellulose, or kaolin; a binder, such as water, gelatin, alcohol, glucose, Gummi Arabicum, or a tragacanth gum; an disintegrant, such as starch, dextrin, or sodium alginate; and a lubricant, such as talc, steric acid, magnesium stearate, or a liquid paraffin. In addition, a dissolution auxiliary for promoting dissolution may be further used.

The pharmaceutical composition according to the present invention may be administered by, for example, oral administration, intranasal administration, intravenous administration, intraperitoneal administration, subcutaneous administration, or topical administration, according to a method that is appropriate for drug administration type and a therapeutical effective dose.

The pharmaceutical composition may also be administered by using other known methods, for example, methods disclosed in Remington's Pharmaceutical Science. For example, injection formulations may be prepared using physiologically suitable buffer solutions, for example, Hank's solution, Ringer's solution, or a saline buffer solution. The solutions may include a suspension, a stabilizer, and/or a dispersant.

Alternatively, the pharmaceutical composition according to the present invention may also be prepared in a powder form, and may be used together with an appropriate carrier, such as sterile water, before administration. Alternatively, the pharmaceutical composition may be prepared in a liposome formulation, a suspension, or a sustained-release formulation. The pharmaceutical composition may be used alone or in combination with surgery, hormone therapy, drug therapy, and a biological reaction controller.

The pharmaceutical composition of the present invention may be used in treating various diseases that require with anti-inflammatory and analgesic effects, such as Bronchitis, Asthma, COPD, -osteo-arthritis, Rheumatic arthritis, degeneratic arthritis and polyarthritis.

An administration dosage and administration time of the pharmaceutical composition may differ according to an age, gender, state, weight, administration path, type of drug used of a subject. A daily administration dose may be in a range of 0.01 ug/kg to 10 g/kg, and may be in a range of 0.01 mg/kg to 100 mg/kg.

All technical terminologies used herein, unless defined otherwise, may be understood as having the same meanings known to one of ordinary skilled in the related art. In addition, although desired methods and samples are introduced in the present specification, methods and samples similar or equivalent thereto may also be included in the scope of the present invention.

The present invention will be described in detail with reference to the examples below. However, the present invention is not limited to these examples.

### Example

### <Reagents and Animals>

A23187 and N-[2-cyclohexyloxy-4-nitrophenyl]methane sulfonamide (NS-398) were obtained from Biomol (Plymouth Meeting, PA); 2-Amino-5,6-dihydro-6-methyl-4H - 1,3-thiazine hydrochloride (AMT) was obtained from Tocris Cookson Ltd. (UK); arachidonic acid (AA, 99%), 3-(4,5-dimethylthiazol-2-yl)-2,5-diphenyltetrazolium bromide (MTT), nordihydroguaiaretic acid (NDGA), prednisolone, indomethacin, aspirin, and lipopolysaccaride (LPS, Escherichia coli 0127:B8) were obtained from Sigma Chem. (St. Louis, MO); and culture reagents such as DMEM and FBS were obtained from Gibco BRL (Grand Island, NY), and a kit for quantifying protein was obtained from Bio-Rad Lab. (Hercules, CA).

Male Sprague-Dawley (SD) rats and ICR mice (4-week old, specific pathogen free) were obtained from Orient-bio (Korea). The laboratory animals were fed with a standard lab. chow (Purina Korea) and water freely, and at least 7 days before the test, the animals were adjusted in an animal breeding room (KNU) at a temperature of 20 to 22°C, at a relative humidity of 40 to 60%, and under a light exposure cycle of 12 hours/12 hours (bright/dark). The animal test design used was approved by the KNU Animal Experiment Committee (KIACUC-09-0012), and this experiment was performed according to a code of ethics speculated in a guide line of Korea Food & Drug Administration regarding protection and use of laboratory animals.

### <Statistical Analysis>

Experimental values were expressed in arithmetic mean ±SD. Statistical significance was determined by performed an ANOVA test.

### <Example 1> Preparation of Extracts from Broussonetia papyrifera, and Lonicera japonica

Broussonetia papyrifera was collected from a southern area of Korea (nearby Andong). The radix bark of Broussonetia papyrifera was dried and the dried radix bark was finely cut and then was subjected to an extraction process using 100% ethanol. An ethanol extract was dried under vacuum conditions and the final extract was dispersed in water and fractionated with ethyl acetate, and then an ethyl acetate fraction was dried. The dried ethyl acetate fraction (EBP) was used in experiments. Papyriflavonol A and broussochalcone A were isolated from EBP according to a method disclosed in Son et al. (2001).

Lonicera japonica was obtained from an oriental medicine market. Dried Lonicera japonica was finely cut and then was subjected to an extraction process using 70% ethanol. An ethanol extract was dried under vacuum conditions and the dried ethanol extract (ELJ) was used in experiments. From the ethanol extract, Loganin and Sweroside were isolated according to a method disclosed in Kawai et al.,1988.

Amounts of prenylated flavonoid (papyriflavonol A and broussochalcone A) in EBP and Iridoids(loganin and sweroside) in ELJ were confirmed by HPLC analysis.

BL is a mixture including EBP and ELJ in a ratio of 1:1 (w/w).

### <Example 2> Rat basophilic leukemia-1 (RBL-1) cell culture and leukotrienes (LT) measurement

RBL-1 cells obtained from American Type Culture Collection (ATCC, Rocksville) were incubated in RPMI 1640 together with 10% FBS, 2 mM glutamine, and 1% antibiotics under 5% CO₂ and at a temperature of 37°C. The cells were placed on 96-well plates for 2 hours, and incubated in advance with a test material for 10 minutes. The test material was dissolved in DMSO and diluted with serum-free DMEM at an appropriate concentration. The final concentration of DMSO was adjusted to be 0.1% (v/v). Cell viability was confirmed by MTT assay according to a method disclosed in Mossman (1983). To activate 5-LOX, A-23187 (ionophore, 3 µM) was added to the cells and the cells were incubated for 15 minutes according to a slightly modified method disclosed in Tries et al. (2002). A medium used was collected and a concentration of cysteinyl leukotrienes (LTC4/D4/E4) produced by 5-LOX was measured using an ELISA kit (Cayman Chem.) according to a method recommended by the manufacturer.

It is known that when RBL-1 cells are activated by A23187 (calcium ionophore), a great amount of cysteinyl-LTs is generated by 5-LOX. In the present experiment, for 15 minutes, an amount of LTC₄/D₄/E₄ produced from RBL-1 cells was 1,109.5 ± 93.6 pg/ml from a basal level of 4.2 ± 1.2 pg/ml for 15 minutes (n=3) to. Under the conditions described above, when a prenylated flavonoid-enriched extract fraction (EBP) from Broussonetia papyrifera was used in an amount of 1 µg/ml, 2 µg/ml, 5 µg/ ml, 10 µg/ml, and 100 µg/ml, a corresponding inhibitory activity was 5%, 24%, 96%, 99% and 99%, respectively. That is, EBP dose-dependently inhibited the production of 5-LOX-mediated LTC₄/D₄/E₄ (FIG. 1). As a result of linear regression analysis, IC₅₀ value was 3.1 µg/ml.

Meanwhile, when ELJ was used in an amount of 10 µg/ml, 20 µg/ml, 50 µg/ml, 100 µg/ml, and 250 µg/ml, a corresponding inhibitory activity was 1%, 6%, 18%, 74% and 99%, respectively. That is, ELJ had a relatively weak inhibitory activity and weakly inhibited the 5-LOX-mediated LTC4/D4/E4 productio (IC₅₀ = 78.7 µg/ml).

A mixture including EBP and ELJ more strongly inhibited the activity of 5-LOX than EBP or ELJ alone. Based on the weight of EBP, IC₅₀ was less than 1 µg/ml. When the inhibitory activity of the mixture was compared to the sum of inhibitory activities of EBP and ELJ, it was confirmed that the inhibitory effect of the mixture against 5-LOX activity was much stronger than when EBP and ELJ were used separately and thus there was a synergy effect stemming from use of the mixture.

For example, when 1 µg/ml of EBP and 4µg/ml of ELJ were used separately, in each case, an inhibitory activity against production of leukotriene (LT) did not occur. However, when EBP and ELJ were used in combination (at a weight ratio of 1:4), an inhibitory activity was unpredictably synergistically increased to 61%. When 2 µg/ml of EBP and 3 µg/ml of ELJ were used separately, an inhibitory activity against production of leukotriene (LT) was 24% and 0%, respectively. However, when EBP and ELJ were used in combination (at a weight ratio of 1:1.5), an inhibitory activity was unpredictably synergistically increased to 92%. When 2.5 µg/ml of EBP and 2.5 µg/ml of ELJ were used in combination (at a weight ratio of 1:1), 5-LOX-mediated LT production was almost completely inhibited and an inhibitory activity was unpredictably synergistically increased to 96%.

The amounts of ELJ used in this experiment corresponded to a level at which a leukotriene production inhibitory activity does not occur when EJL was used alone. However, when ELJ was used together with EBP, 5-LOX inhibitory activity of EBP was substantially enhanced by addition of ELJ and the mixture including EBP and ELJ showed a very high inhibitory effect. In addition, an additional experiment was performed using a mixture (BL) including EBP and ELJ at a weight ratio of 1:1.

Nordihydroguaiaretic acid (NDGA) was used as a comparative compound and also had a strong 5-LOX inhibitory activity (99 % inhibitory activity at 1 µM).

### <Example 3> Effects on production of PGE2 in RAW 264.7 cells

This experiment was performed to identify effects of extracts from Broussonetia papyrifera and Lonicerajaponica on production of PGE₂, which is a mediator of an inflammatory reaction.

RAW 264.7 cells obtained from American Type Culture Collection (ATCC, Rocksville) was incubated in DMEM supplemented with 10% FBS and 1% antibiotics (100 U/ml of penicillin and 100 µg/ml of streptomycin) under 5% CO₂ at a temperature of 37°C. The cells were activated with lipopolysaccaride (LPS) according to a method disclosed in Chi et al. (2001). The cells were loaded to 96-well plates (2x10⁵ cells/well) and pre-incubated for 2 hours, and then a test material and LPS (1 µg/ml) were added thereto and the resultant medium was incubated for 24 hours unless defined otherwise. A concentration of PGE2 in the medium was measured by using an ELISA kit for PGE2 (Cayman Chem. Co.) according to a method recommended by the manufacturer.

EBP and BL statistically significantly inhibited production of COX-2-catalyzed PGE2 from RAW 264.7 cells which are LPS-treated mouse microphage cell lines, at a concentration of 50 ug/ml (FIG. 2). IC₅₀ values of EBP and BL with respect to production of PGE₂ were 21.4 µg/ml and 29.0 µg/ml, respectively.

### <Example 4> Effects on arachidonic acid (AA)-induced mouse edema

AA-induced mouse ear edema assay was used to identify an anti-inflammatory activity in vivo in anti-inflammatory animal model. According to a method disclosed in Kim et al. (1993), 2% AA acetone solution was applied to an ear of a mouse (20 µl/ ear), and after one hour, a thickness of the ear was measured using a dial thickness gauge (Mitutoyo, Japan). One hour before the AA treatment, a test material dissolved in a mixture including DMSO and water at a mixture ratio of 1:1 was orally administered to the mouse (0.1 ml/mouse). Each group consisted of 5 mice, and measurement values were represented by arithmetic mean± S.D (a of Table-1). b of Table 1 indicates an inhibitory activity % compared to the control group treated with AA. * indicates a significant difference of P<0.05 compared to the AA-treated control group.

Table 1

**[Table 1]**

| Compounds | Dose(mg/kg) | Ear thickness increased, mm |
|---|---|---|
| AA-treated | - | 0.070±0.010^{a}(-)^{b)} |
| Indomethacin | 20 | 0.021±0.013* (70.0) |
| EBP | 50 | 0.050±0.037 (28.6) |
| EBP | 100 | 0.032±0.015*(54.3) |
| EBP | 200 | 0.038±0.023*(45.7) |
| ELJ | 50 | 0.090±0.034 (-) |
| ELJ | 100 | 0.054±0.017 (22.9) |
| ELJ | 200 | 0.053±0.025 (25.0) |
| BL | 50 | 0.064±0.029 (8.6) |
| BL | 100 | 0.046±0.015*(34.3) |
| BL | 200 | 0.028±0.008*(60.0) |
| BL | 400 | 0.015±0.013*(78.6) |

### [Inhibition of AA-induced mouse ear edema]

A negative control group that was not treated with AA had an ear thickness increase of 0.003 ± 0.001 mm. When 50 - 200 mg/kg of EBP and ELJ were orally administered, edema was inhibited, and EBP had a stronger edema inhibitory effect than ELJ (Tablel). When BL, which is a mixture including EBP and ELJ at a weight ratio of 1:1, was administered, compared to when EBP and ELJ were separately administered, an inflammation suppression effect was synergistically increased.

For example, when 50 mg/kg of EBP was administered, an inhibitory effect on the ear edema was 28.6%, and when 50 mg/kg of ELJ was administered, no inhibitory effect occurred. However, BL 100 mg/kg of BL including 50 mg/kg of EBP and 50 mg/kg of ELJ had an inhibitory effect of 34.4%. That is, when BL was administered, an unpredictable synergic effect occurred. In addition, when 200 mg/kg of EBP was administered, the inhibitory activity against the ear edema was 45.7%, and when 200 mg/kg of ELJ was administered, the inhibitory activity against the ear edema was 25.0%.

In addition, when 200 mg/kg of BL was administered, the inhibitory activity against the ear edema was 60.0%. That is, groups treated with BL had the highest inhibitory effect. Also, when BL 400 mg/kg of BL including 200 mg/kg of EBP and 200 mg/kg of ELJ were used, the inhibitory activity was 78.6% that is greater than the sum of the inhibitory activities of EBP and ELJ. This result means that combination of EBP and ELJ results in a synergic effect. In particular, the inhibitory activity of the 400mg/kg of BL mixture is greater than 70% inhibitory activity of Indomethacin (20 mg/kg).

### <Example 5> Effects on λ-Carageenan (CGN)-induced mouse paw edema

Mouse CGN-induced paw edema assay was performed to measure an anti-inflammatory activity according to a slightly modified method disclosed in Winter et al. (1962). A test material was orally administered to a mouse, and after 1 hour, 1% CGN (w/v) solution (0.05 ml/paw) prepared by dissolving CGN in a pyrogen-free sterilized saline was injected to a paw. After 5 hours, a volume of the paw was measured using plenthysmometer (Ugo Basil, Italy). If the volume was greater than that before the addition of CGN, it was deemed that edema developed.

The test material was dissolved in a mixture including DMSO and water at a mixture ratio of 1:1 and then was orally administered. A volume of a negative control group which was not treated with CGN was 0.149± 0.025 ml. Each group consisted of 5 mice, and measurement values were represented by arithmetic mean ± SD (a of Table 2). b of Table 2 indicates an inhibitory activity % compared to the control group treated with CGN. * indicates a significant difference of P<0.05 compared to the CGN -treated control group.

Table 2

**[Table 2]**

| Compounds | Dose(mg/kg) | Paw volume increased,ml |
|---|---|---|
| CGN-treated | - | 0.152±0.022^{a)} (-)^{b)} |
| Indomethacin | 20 | 0.116±0.039 (23.7) |
| Indomethacin | 100 | 0.084+0.019* (44.7) |
| Prednisolone | 20 | 0.076±0.011* (50.0) |
| EBP | 200 | 0.116±0.045 (23.7) |
| ELJ | 200 | 0.134±0.047 (11.8) |
| BL | 50 | 0.136±0.030 (10.5) |
| BL | 100 | 0.120±0.047 (21.1) |
| BL | 200 | 0.100±0.020*(34.2) |
| BL | 400 | 0.080±0.050* (47.4) |

### [Inhibition of carrageenan(CGN)-induced mouse paws edema]

Regarding CGN-induced paw edema, when 50 to 400 mg/kg of BL was orally administered, the group treated with BL had a dose-dependent inhibitory activity (Table 2). When 200 mg/kg of EBP and 200 mg/kg of ELJ were administered separately, an inhibitory activity of EBP was 23.7% and an inhibitory activity of ELJ of 11.8% and the sum of the inhibitory activities was 35.5%. On the other hand, when 400 mg/kg of BL including 200 mg/kg of EBP and 200 mg/kg of ELJ was administered, an inhibitory activity of BL was 47.4%. Thus, it is confirmed that use of EBP and ELJ in combination leads to a synergic inhibitory effect.

### <Example 6> Acetic acid-induced writhing test on mice

In order to measure an antinociceptive activity, 1% acetic acid (100 µl) was intraperitoenally injected to a mouse. 10 minutes after the injection of the acetic acid, the number of writhings was counted for 10 minutes. Before the injection of the acetic acid, a test material dissolved in a mixture including DMSO and water at a ratio of 1:1 was orally administered (0.1 ml/mouse).

Each group consisted of 6 mice. Measurement values were represented by arithmetic mean ± SD (a of Table 3). b of Table 3 indicates an inhibitory activity % compared to the control group treated with the acetic acid. * indicates a significant difference of P<0.05 compared to the acetic acid -treated control group.

Table 3

**[Table 3]**

| Compounds | Dose(mg/kg) | Numbers of writings |
|---|---|---|
| Exp.1 | | |
| Acetic acid-treated | - | 14.5±6.4^{a)} (-)^{b)} |
| Aspirin | 100 | 5.7±3.8*(60.9) |
| EBP | 50 | 12.2±5.2 (16.1) |
| EBP | 100 | 8.5±3.6 (41.4) |
| EBP | 200 | 7.5±5.2 (48.3) |
| ELJ | 100 | 8.2±5.1 (43.7) |
| ELJ | 200 | 5.8±4.4 (59.8) |
| BL | 50 | 8.8±5.9 (39.1) |
| BL | 100 | 7.7±4.0 (47.1) |
| BL | 200 | 7.3±5.8 (49.4) |
| BL | 400 | 4.5±3.3* (69.0) |
| Exp.2 | | |
| Acetic acid-treated | - | 17.8±6.1 (-) |
| Aspirin | 100 | 9.6±3.2* (46.1) |
| BL | 100 | 11.6±6.4 (34.8) |
| BL | 200 | 10.8±5.5 (39.3) |
| BL | 400 | 8.6±4.3* (51.7) |

### [Inhibition of acetic acid-induced writhings in mice]

As a result of the antinociceptive activity assay, when 50 to 400 mg/kg of ELJ was orally administered, an antinociceptive activity of ELJ was greater than that of EBP, and BL strongly and dose-dependently inhibited the acetic acid-induced writhing in the mouse (Table 3).

It had been reported that that several parts of Broussonetia papyrifera have antinociceptive and anti-inflammatory activities (Lin et al., 2008). When 600 to 2000 mg/kg of an ethanol extract of a radix, leave, or fruit of Broussonetia papyrifera was orally administered, acetic acid-induced writhing was inhibited in vivo in mice. Compared to the results, the composition according to the present invention had stronger antinociceptive and anti-inflammatory activities in vivo at a concentration of 50 to 400 mg/kg.

Various types of formulations were prepared with the composition including extracts from Broussonetia papyrifera and Lonicera japonica as an effective component.

### Preparation Example 1: Preparation of Tablet

Prenylated flavonoid-enriched extract from Broussonetia papyrifera 100 mg
Ethanol extract from Lonicera japonica 100 mg
Lactose 50 mg
Starch 10 mg
Stearic Acid Magnesium appropriate amount
The components were mixed and tableted according to a conventional tablet preparation method, thereby formulating a tablet.

### Preparation Example 2: Preparation of Powder

Prenylated flavonoid-enriched extract from Broussonetia papyrifera 100mg
Ethanol extract from Lonicera japonica 400mg
Lactose 30 mg
Starch 20 mg
Stearic acid magnesium appropriate amount
The components were thoroughly mixed and a polyethylene-coated slice was filled with the mixture and sealed, thereby preparing a powder formulation.

### Preparation Example 3: Preparation of Capsule

Prenylated flavonoid-enriched extract from Broussonetia papyrifera 200 mg
Ethanol extract from Lonicera japonica 100 mg
Lactose 150 mg
Starch 28 mg
Talc 2 mg
Stearic acid magnesium appropriate amount
The components were mixed and a gelatin soft capsule was filled with the mixture according to a conventional capsule preparation method, thereby preparing a capsule.

### Preparation Example 4: Preparation of Soft Capsule

Prenylated flavonoid-enriched extract from Broussonetia papyrifera 200 mg
Ethanol extract from Lonicera japonica 200 mg
Polyethyleneglycol 400 400 mg
Concentrated Glycerin 55 mg
Purified water 35 mg
Polyethyleneglycol and concentrated glycerin were mixed and then purified water was added thereto. Flavone was added to the mixture while the temperature was maintained at about 60°C and the resultant mixture was uniformly stirred by an agitator at a rotational rate of about 1,500 rpm. The mixed solution was cooled to room temperature while slowly stirring, and bubbles that were generated thereby were removed using a vacuum pump, thereby preparing a content for a soft capsule.

A cover film of the soft capsule was prepared using, per one capsule, 132 mg of gelatin, 52 mg of concentrate glycerin, 6 mg of 70% disorbitol solution, an appropriate amount of ethyl vanillin as a fragrance ingredient, and carnauba as a coating substrate according to a conventional preparation method.

### <References>

Cheng Z-J, Lin C-N, Hwang T-L, Teng C-M. Broussochalcone A, a potent antioxidant and effective suppressor of inducible nitric oxide synthase in lipopolysaccharide-activated macrophages. Biochem. Pharmacol. 61, 939-946 (2001).
Damas J, Bourdon V, Remacle-Volon G, Adam A. Kinins and peritoneal exudates induced by caffageenin and zymosan in rats. Br. J. Pharmacol. 101, 418-422 (1990).
Editorial Committee of Zhong Hua Ben Cao of State Administration of Traditional Chinese Medicine of People's Republic of China, 1999. Zhong Hua Ben Cao vol. 2, Shanghai Science and Technology Publishing Co., Shanghai, pp. 473-474.
Hwang JH, Lee BM. Inhibitory effects of plant extracts on tyrosinase, L-DOPA oxidation, and melanin synthesis. J. Toxicol. Environ. Health A 70, 393-407 (2007).
Holsapplae MP, Yim GK. Therapeutic reduction of ongoing carrageenin-induced inflammation by lipoxygenase, but not cyclooxygenase inhibitors. Inflammation 8, 223-230 (1984).
Inoue H, Mori T, Koshihara Y. Sulfidopeptide-leukottienes are major mediators of arachidonic acid-induced mouse ear edema. Prostaglandins 36, 731-739 (1988).
Kawai H, Kutoyanagi M, Ueno A. Iridoid glucosides from Lonicera japonica Thunb. Chem. Pharm. Bull. 36, 3664-3666 (1988).
Kim HK, Namgoong SY, Kim HP. Anti-inflammatory activity of flavonoids: Mice ear edema inhibition. Arch. Pharm. Res. 16, 18-24 (1993).
Kwak WJ, Han CK, Chang HW, Kim HP, Kang SS, Son KH. Loniceroside C, an anti-inflammatory saponin from Lonicerajaponica. Chem. Pharm. Bull. 51, 333-335 (2003).
Lee SJ, Shin EJ, Son KH, Chang HW, Kang SS, Kim HP. Anti-inflammatory activity of the major constituents of Lonicera japonica. Arch. Pharm. Res. 18, 133-135 (1995).
Lee SJ, Son KH, Chang HW, Kang SS, Kim HP. Anti-inflammatory activity of Lonicerajaponica. Phytother. Res. 12,445-447 (1998).
Lin LW, Chen HY, Wu CR, Liao PM, Lin YY, Hsieh MT, Ching H. Comparison with various parts of Broussonetia papyriferaas to the antinociceptive and antiinflammatory activities in rodents. Biosci. Biotechnol. Biochem. 72, 2377-2384 (2008).
Mossman, T., Rapid colorimetric assay for cellular growth and survival: application to proliferation and cytotoxic assays. J. Immunol. Methods, 65, 55-63 (1983).
Qian ZM, Li HJ, Li P, Chen J, Tang D. Simultaneous quantification of seven bioactive components in Caulis Lonicera japonicae by high performance liquid chromatography. Biomed. Chromatogr. 21, 649-654 (2007).
Shougakukan, "The dictionary of Chinese drugs," vol. 3, Shanghai Science and Technologic Publishers and Shougakukan, Tokyo, 1985, pp. 2027-2029.
Son KH, Kwon SJ, Chang HW, Kim HP, Kang SS. Papyriflavonol A, a new prenylated flavonoid from Broussonetia papyrifera. Fitoterapia 72, 456-458 (2001).
Tries S, Neupert W, Laufer S. The mechanism of action of the new anti-inflammatory compound ML3000: inhibition of 5-LOX and COX-1/2. Inflamm. Res. 51, 135-143 (2002).
Vinegar R, Schreiber W, Hugo R. Biphasic development of carrageenin edema in rats. J. Pharmacol. Exp. Ther. 166, 96-103 (1969).
Wang J-P, Tsao L-T, Raung S-L, Lin C-N. Investigation of the inhibitory effect of broussochalcone A on respiratory burst in neutrophils. Eur. J. Pharmacol. 320, 201-208 (1997).
Winter, C. A., Risley, E. A., and Nuss, G. W., Carrageenan-induced edema in the hindpaw on the rat as an assay for anti-inflammatory drugs. Proc. Soc. Exp. Biol. Med., 111, 544-547 (1962).

### Industrial Applicability

According to the present invention, a composition including extracts from Broussonetia papyrifera and Lonicera japonica as effective components may be used as an effective anti-inflammatory agent, antiphlogistic agent, or analgesic agent because when the extracts are used in combination, better anti-inflammatory and antinociceptive effects are obtained than they are used separately.

## Claims

1. A pharmaceutical composition for use in preventing, alleviating, or treating inflammation and/or pains, the pharmaceutical composition comprising:
as an effective component, a first material selected from the group consisting of a fraction of the extract from Broussonetia papyrifera, and a pharmaceutically acceptable salt thereof;
as an effective component, a second material selected from the group consisting of an extract from Lonicera japonica and a pharmaceutically acceptable salt thereof; and a pharmaceutically acceptable carrier,
wherein the fraction of the extract from Broussonetia papyrifera is a prenylated flavonoid- enriched extract prepared by extracting Broussonetia papyrifera with ethanol, drying the extract under vacuum condition, and fractionating the extract using ethyl acetate, and the extract from Lonicera japonica is an ethanol extract.

2. A pharmaceutical composition according to the use of claim 1, wherein the disease prevented or treated requires with anti-inflammatory and analgesic effects and is selected from the group consisting of Bronchitis, Asthma, COPD, osteo-arthritis, Rheumatic arthritis, degeneratic arthritis, and polyarthritis.

3. The pharmaceutical composition according to the use of any one of claims 1 to 2, wherein a weight ratio of the fraction of the extract from Broussonetia papyrifera to the extract from Lonicera japonica is 1: 0.1 to 10.

4. The pharmaceutical composition according to the use of any one of claims 1 to 2, wherein the weight ratio of the fraction of the extract from Broussonetia papyrifera to the extract from Lonicera japonica is 1:1.

5. A pharmaceutical formulation prepared using the pharmaceutical composition according to the use of any one of claims 1 to 4, wherein the pharmaceutical formulation is selected from the group consisting of a formulation for oral administration, a formulation adaptable for a mucous membrane, an injection formulation, an inhalant formulation, and an external formulation.

6. The pharmaceutical formulation of claim 5, wherein the formulation for oral administration is selected horn the group consisting of hard and sot capsule, tablet, suspension, syrup, powder, sustained-release formulation, enteric coated formulation, granule, oleosaccharum, fine granule, pill, extract, liquid formulation, aromatic water formulation, emulsion, elixir, fluid extracts, precipitation formulation, tincture and spirits.

## Patentansprüche

1. Pharmazeutische Zusammensetzung zur Verwendung beim Verhindern, Lindern oder Behandeln einer Entzündung und/oder von Schmerzen, wobei die pharmazeutische Zusammensetzung umfasst:
als wirksamen Bestandteil, ein erstes Material, das aus der Gruppe bestehend aus einer Fraktion des Extrakts aus Broussonetia papyrifera und einem pharmazeutisch akzeptablen Salz davon ausgewählt ist;
als wirksamen Bestandteil, ein zweites Material, das aus der Gruppe bestehend aus einem Extrakt aus Lonicera japonica und einem pharmazeutisch akzeptablen Salz davon ausgewählt ist; und
einen pharmazeutisch akzeptablen Träger,
wobei die Fraktion des Extrakts aus Broussonetia papyrifera ein prenylierter flavonoidangereicherter Extrakt ist, der durch Extrahieren von Broussonetia papyrifera mit Ethanol, Trocknen des Extrakts unter Vakuumbedingung und Fraktionieren des Extrakts unter Verwendung von Ethylacetat hergestellt wird, und der Extrakt aus Lonicerajaponica ein Ethanolextrakt ist.

2. Pharmazeutische Zusammensetzung gemäß der Verwendung nach Anspruch 1, wobei die Erkrankung, die verhindert oder behandelt wird, antientzündliche und schmerzstillende Wirkungen erfordert und aus der Gruppe bestehend aus Bronchitis, Asthma, COPD, Osteoarthritis, rheumatoider Arthritis, degenerativer Arthritis und Polyarthritis ausgewählt ist.

3. Pharmazeutische Zusammensetzung gemäß der Verwendung nach einem der Ansprüche 1 bis 2, wobei ein Gewichtsverhältnis der Fraktion des Extrakts aus Broussonetia papyrifera zu dem Extrakt aus Lonicera japonica 1 : 0,1 bis 10 beträgt.

4. Pharmazeutische Zusammensetzung gemäß der Verwendung nach einem der Ansprüche 1 bis 2, wobei das Gewichtsverhältnis der Fraktion des Extrakts aus Broussonetia papyrifera zu dem Extrakt aus Lonicera japonica 1:1 beträgt.

5. Pharmazeutische Formulierung, die unter Verwendung der pharmazeutischen Zusammensetzung gemäß der Verwendung nach einem der Ansprüche 1 bis 4 hergestellt wird, wobei die pharmazeutische Formulierung aus der Gruppe bestehend aus einer Formulierung zur oralen Verabreichung, einer Formulierung, die für eine Schleimhaut anpassbar ist, einer Injektionsformulierung, einer Inhalationsmittelformulierung und einer externen Formulierung ausgewählt ist.

6. Pharmazeutische Formulierung nach Anspruch 5, wobei die Formulierung zur oralen Verabreichung aus der Gruppe bestehend aus Hart- und Weichkapsel, Tablette, Suspension, Sirup, Pulver, Retard-Formulierung, Formulierung mit magensaftresistenter Beschichtung, Körnchen, Oleo saccharum, feinem Körnchen, Pille, Extrakt, flüssiger Formulierung, Formulierung mit aromatischem Wasser, Emulsion, Elixier, Fluidextrakten, Abscheidungsformulierung, Tinktur und Spirituosen ausgewählt ist.

## Revendications

1. Composition pharmaceutique pour utilisation dans la prévention, le soulagement ou le traitement d'inflammations et/ou de douleurs, la composition pharmaceutique comprenant :
comme composant efficace, un premier matériau sélectionné parmi le groupe se composant de : une fraction d'un extrait de Broussonetia papyrifera, et un sel de celui-ci acceptable d'un point de vue pharmaceutique ;
comme composant efficace, un deuxième matériau sélectionné parmi le groupe se composant de : une fraction d'un extrait de Lonicera japonica, et un sel de celui-ci acceptable d'un point de vue pharmaceutique ; et un support pharmaceutiquement acceptable.
la fraction de l'extrait de Broussonetia papyrifera étant un extrait enrichi aux flavonoïdes prénylés préparé par extraction de Broussonetia papyrifera avec de l'éthanol, séchage de l'extrait dans des conditions sous vide et fractionnement de l'extrait en utilisant de l'acétate d'éthyle, et l'extrait de Lonicera japonica étant un extrait éthanolique.

2. Composition pharmaceutique selon l'utilisation de la revendication 1, la maladie prévenue ou traitée nécessitant des effets anti-inflammatoires et analgésiques et étant sélectionnée parmi le groupe se composant de : bronchite, asthme, BPCO, ostéoarthrite, polyarthrite rhumatoïde, arthrite dégénérative et polyarthrite.

3. La composition pharmaceutique selon l'utilisation de l'une quelconque des revendications 1 à 2, un rapport de poids de la fraction de l'extrait de Broussonetia papyrifera avec l'extrait de Lonicerajaponica étant de 1: 0,1 à 10.

4. La composition pharmaceutique selon l'utilisation de l'une quelconque des revendications 1 à 2, le rapport de poids de la fraction de l'extrait de Broussonetia papyrifera avec l'extrait de Lonicera japonica étant de 1:1.

5. Préparation pharmaceutique préparée en utilisant la composition pharmaceutique selon l'utilisation de l'une quelconque des revendications 1 à 4, la préparation pharmaceutique étant sélectionnée parmi le groupe se composant de : une préparation pour administration orale, une préparation adaptable pour une membrane muqueuse, une préparation pour injection, une préparation pour inhalation et une préparation externe.

6. La préparation pharmaceutique de la revendication 5, la préparation pour l'administration orale étant sélectionnée parmi le groupe se composant de : capsule dure et molle, comprimé, suspension, sirop, poudre, préparation à libération prolongée, préparation à enrobage entérique, granulé, oleosaccharum, granulé fin, pilule, extrait, préparation liquide, préparation à l'eau aromatique, émulsion, élixir, extraits fluides, préparation par précipitation, teinture et spiritueux.
